# EUROPEAN PATENT APPLICATION

(11) **EP 4 838 224 A1**
(43) Date of publication of application: **11.11.2026**
(21) Application number: 26176549.9
(22) Date of filing: 05.05.2026
(51) Int. Cl.: G01N 33/543, C07K 16/44, G01N 33/74

(54) **METHOD FOR DETECTING SMALL MOLECULE**

(30) Priority: 07.05.2025 CN 202510585347
(71) Applicant: Shenzhen New Industries Biomedical Engineering Co., Ltd., Shenzhen, Guangdong 518122 (CN)
(72) Inventor: GAO, Li, Shenzhen 518122 (CN); CHEN, Ni, Shenzhen 518122 (CN); REN, Jing, Shenzhen 518122 (CN); LI, Xiaomin, Shenzhen 518122 (CN); DU, Kai, Shenzhen 518122 (CN); HE, Haihua, Shenzhen 518122 (CN); LI, Tinghua, Shenzhen 518122 (CN); RAO, Wei, Shenzhen 518122 (CN)
(74) Representative: Lapienis, Juozas

(57) **Abstract**

The present invention relates to a method for detecting a small molecule. The method includes forming a multi-component complex comprising a "first complex antibody - complex of small molecules/small molecule binding partner - second complex antibody", and detecting the small molecule in a sample by detecting the multi-component complex; the small molecule binding partner is capable of specifically binding to the small molecules and has at least two antigen-binding sites, and is capable of binding to at least two small molecules to obtain the complex of small molecules/small molecule binding partner, and forming at least two new conformational epitopes distinct from the small molecule in a free state and the small molecule binding partner in a free state; the first complex antibody and the second complex antibody each specifically bind to at least one new conformational epitope, and neither the first complex antibody nor the second complex antibody is an antibody against the small molecule antigen or a specific antibody against the small molecule binding partner.

## Description

### TECHNICAL FIELD

The present invention generally relates to the technical field of detecting small molecules, and specifically, relates to a method for detecting a small molecule.

### BACKGROUND ART

Due to their simple chemical structures and small molecular weights, small molecule substrates have problems such as having a single antigenic recognition epitope or having a plurality of antigenic epitopes, but overlapping among the epitopes or the epitopes being embedded. Currently, rapid detection technologies for detecting small molecule substances are mostly based on a competitive immunoassay (abbreviated as "competitive method") relying on an antigen-antibody specific recognition mechanism. However, the competitive method achieve sensitivity at an ng/L level only when an antibody affinity constant exceeds 10¹¹ L/mol, and an antibody with an ultra-high affinity is difficult to prepare. Therefore, developing highly sensitive assay kits for competitive method is challenging, and it is difficult to distinguish negative samples in a trace detection. Furthermore, the competitive method recognizes an analyte to be tested through only one site, making them prone to cross-reactivity, thereby leading to a problem of false positives.

Therefore, currently, immunoassay methods for small molecule substrates still require further research.

### SUMMARY

The present invention relates to a method for detecting a small molecule, including: forming a multi-component complex including a "first complex antibody - complex of small molecules/small molecule binding partner - second complex antibody", and detecting the small molecule in a sample by detecting the multi-component complex,
wherein a molecular weight of the small molecule is less than 5,000 Daltons; and
the small molecule binding partner is capable of specifically binding to the small molecules and has at least two antigen-binding sites, and is capable of binding to at least two small molecules to obtain the complex of small molecules/small molecule binding partner, and forming at least two new conformational epitopes distinct from the small molecule in a free state and the small molecule binding partner in a free state; and the first complex antibody and the second complex antibody each specifically bind to at least one new conformational epitope, and neither the first complex antibody nor the second complex antibody is an antibody against the small molecule antigen or a specific antibody against the small molecule binding partner.

According to another aspect of the present invention, the present invention relates to a kit, including the first complex antibody, the small molecule binding partner, and the second complex antibody as defined above.

The new detection method can significantly improve sensitivity and specificity for detecting a small molecule and avoid the Hook effect.

### BRIEF DESCRIPTION OF DRAWINGS

To more clearly illustrate specific embodiments of the present invention or the technical solutions in the prior art, drawings that need to be used in description of the specific embodiments or the prior art will be briefly introduced below. Obviously, the drawings of the description below are some embodiments of the present invention. For those ordinarily skilled in the art, other drawings can be obtained based on these drawings without making any inventive efforts.
FIG. 1 is a structural diagram of the multi-component complex of the present invention; and
FIG. 2 is a correlation curve between a sandwich method and a competitive method for detecting cortisol provided by an embodiment of the present invention.

### DETAILED DESCRIPTION OF EMBODIMENTS

References to embodiments of the present invention will be provided in detail below, and one or more of examples are described below. Each example is provided as an explanation rather than a limitation on the present invention. In fact, it is obvious for those skilled in the art that various modifications and variations can be made to the present invention without deviating from the scope or spirit of the present invention. For example, a feature illustrated or described as part of an embodiment can be used in another embodiment to provide a further embodiment.

Unless otherwise specified, meanings of all terms used to disclose the present invention (including technical and scientific terms) are the same as those commonly understood by those ordinarily skilled in the art to which the present invention belongs. Through further guidance, subsequent definitions are provided to better understand teachings of the present invention. The terms used in a specification of the present invention are only for a purpose of describing specific embodiments and are not intended to limit the present invention.

In the present invention, unless otherwise specified, scientific and technical terms used herein have the meanings commonly understood by those skilled in the art. Furthermore, terms and operation procedures in laboratory related to protein and nucleic acid chemistry, molecular biology, cell and tissue culture, microbiology, and immunology used herein are all widely used terms and routine procedures in corresponding fields. In addition, to better understand the present invention, definitions and explanations of relevant terms are provided below.

Terms "containing", "comprising", and "including" used in the present invention are synonyms and are inclusive or open-ended, not excluding additional or uncited members, elements, or method steps.

In the present invention, a numerical range represented by endpoints includes all values and fractions included within the range, as well as cited endpoints.

The present invention relates to concentration values, and a meaning thereof includes fluctuations within a certain range. For example, fluctuations are allowed within a corresponding precision range. For example, 2% can allow fluctuations within a range of ±0.1%. For numerical values that are relatively large or do not require excessively fine control, a meaning thereof is further allowed to include greater fluctuations. For example, 100 mM can allow fluctuations within ranges of ±1%, ±2%, ±5%, etc.

As used in the present invention, unless otherwise specified, singular-form articles "a", "an", and "the" include plural referents.

In the present invention, descriptions involving "a plurality of", "a variety of", etc., refer to a quantity greater than or equal to 2 unless otherwise specified.

In the present invention, technical features described in an open-ended manner include both closed-ended technical solutions composed of the listed features and open-ended technical solutions including the listed features.

In the present invention, "preferred", "better", "more preferable", and "suitable" are merely used to describe embodiments or examples with better effects, and it should be understood that they do not constitute a limitation on the scope of protection of the present invention. In the present invention, "optionally", "optional", and "option" mean something that is dispensable, that is, selected from either of the two parallel solutions of "with" or "without". If there are a plurality of "optional" statements in a technical solution, unless otherwise specified and without contradiction or mutual restriction relationship, each "optional" statement is independent.

In the present invention, a term "detection" refers to qualitative detection, quantitative detection, or semi-quantitative detection for small molecules.

In the present invention, a "sample" includes both a biological material and a non-biological material. The non-biological material includes, for example, an environmental sample (such as water, e.g., domestic water, river water, seawater, and well water; soil; food; and clothing). A term "biological material" used herein generally refers to a biological material suspected of containing small molecule analytes. The biological material can be derived from any biological source, such as physiological fluids including blood, plasma, serum, tissue fluid, saliva, lens fluid, cerebrospinal fluid, sweat, urine, milk, ascites, mucus, nasal discharge, sputum, synovial fluid, peritoneal fluid, vaginal fluid, menstrual blood, amniotic fluid, semen, etc. A sample can be used directly after being obtained from a biological or non-biological source, or can be used after a pretreatment to improve characteristics of the sample. For example, the pretreatment can include preparing plasma from blood, diluting viscous liquids, etc. Pretreatment methods further include filtration, precipitation, dilution, dissociation of a small molecule from a binding state to a free state, distillation, mixing, concentration, inactivation of interfering components, addition of reagents, bacteriolysis, etc. In addition, it is advantageous to transform a solid test sample into a liquid medium or release analytes.

In the present invention, a term "small molecule" refers to a molecule with a molecular weight less than 5,000 Daltons. The small molecule can have a molecular weight less than 3,000 Daltons, can have a molecular weight less than 2,000 Daltons, and can have a molecular weight less than 1,500 Daltons, or a molecular weight less than 1,200 Daltons, or a molecular weight less than 1,000 Daltons. In addition, unless specifically emphasized as a complex, the "small molecule" according to the context generally refers to a state that has not formed a complex with an anti-small-molecule antibody. In addition, for some types of small molecules, such as hormones, unless specifically emphasized, the "small molecule" generally does not distinguish between a free state and a binding state. Herein, a "free state" and a "binding state" refer to an unbinding/binding state of the small molecule to other substances (such as plasma proteins) in a sample to be tested. If the binding state interferes with the detection of the small molecule, those skilled in the art can treat it using commonly known methods to convert it into a small molecule in a free state before detecting.

In the present invention, a term "small molecule binding partner" refers to a substance capable of specifically binding to the small molecule and having at least two antigen-binding sites, and capable of binding to at least two small molecules to obtain the complex of small molecules/small molecule binding partner and forming at least two new conformational epitopes. A typical structure thereof is an anti-small-molecule antibody. In addition, it can further be some binding proteins in a form of multimer, and an example of each monomer thereof can be at least a partial variable region of an anti-small-molecule antibody, or a substance, such as receptor/ligand, etc., that specifically binds to the small molecule.

In the present invention, unless otherwise specified, a term "antibody" includes a complete antibody and an antibody fragment. A term "antibody fragment" includes antigen-compound binding fragments of these antibodies, including Fab, F(ab')₂, Fd, Fv, scFv, and the smallest recognition unit of antibodies, as well as single-chain derivatives of these antibodies and fragments, such as scFv-Fc. A type of the antibody can be selected from IgG1, IgG2, IgG3, IgG4, IgA, IgM, IgE, and IgD. In addition, the term "antibody" includes both naturally occurring antibodies and non-naturally occurring antibodies, including, such as, chimeric and humanized antibodies, and human antibodies, as well as related synthetic isoforms. The term "antibody" can be used interchangeably with "immunoglobulin". A species source of the antibody includes, but is not limited to, cattle, horses, dairy cows, pigs, sheep, goats, rats, mice, dogs, cats, rabbits, camels, donkeys, deer, mink, chicks, ducks, geese, turkeys, fighting cocks, etc. Specifically, a term "anti-small-molecule antibody" refers to an antibody specifically binding to a small molecule to be tested, including a complete antibody and an antibody fragment that contain at least two antigen-binding sites. The two antigen-binding sites thereof refer to at least two variable regions on the anti-small-molecule antibody that specifically bind to the small molecule to be tested. Amino acid sequences of the two variable regions can be identical or different, as long as they are capable of specifically binding to the small molecule to be detected.

A term "new conformational epitope" used in the present invention refers to a new conformational epitope obtained after the small molecule to be tested binds to the small molecule binding partner to form a complex. The conformational epitope is selected from a linker moiety between the small molecule to be tested and the small molecule binding partner, or from a region where the small molecule binding partner undergoes a conformational change (in the case of the small molecule binding partner being an anti-small-molecule antibody, it is usually located in a variable region such as a CDR region or a linking position between the CDR region and a FR region). Since the small molecule binding partner has at least two antigen-binding sites, there must be at least two new conformational epitopes in the complex of small molecules/small molecule binding partner formed after their binding. These two new conformational epitopes can be identical or different.

A term "anti-complex antibody" as used in the present invention refers to an antibody capable of specifically binding to the above new conformational epitopes, which does not bind or weakly binds to the small molecule binding partner or the small molecule to be tested in a free state.

In the present invention, a term "complementarity-determining region" or "CDR" refers to a highly variable region of heavy chains and light chains of an immunoglobulin, as defined by Kabat *et al.* (Kabat et al., Sequences of proteins of immunological interest, 5th Ed., US Department of Health and Human Services, NIH, 1991, and later versions). There are three heavy chain CDRs and three light chain CDRs. Herein, depending on the context, the terms "CDR" and "CDRs" are used to refer to regions containing one or more, or even all regions, of the major amino acid residues that contribute to binding affinity of an antibody to the antigens or epitopes it recognizes. In another specific embodiment, a CDR region or CDR refers to the highly variable region of the heavy chains and the light chains of an immunoglobulin as defined by Kabat.

All the documents mentioned in the present invention are hereby incorporated by reference into the present invention, as if each individual document was specifically and individually indicated to be incorporated by reference. Unless conflicting with an inventive purpose and/or technical solution of the present invention, the references cited in the present invention are incorporated herein by reference in entirety and for all purposes. When the present invention involves citing references, definitions of relevant technical features, terms, nouns, phrases, etc., in the cited references are also incorporated herein by reference. When the present invention involves citing references, examples and preferred embodiments of the relevant technical features cited are also incorporated herein by reference in the present invention, provided that the present invention can be implemented. It should be understood that when the cited content conflicts with description of the present invention, the present invention shall prevail or modifications shall be made adaptively according to the description of the present invention.

The first aspect of the present invention relates to a method for detecting a small molecule, including: forming a multi-component complex including a "first complex antibody - complex of small molecules/small molecule binding partner - second complex antibody", and detecting the small molecule in a sample by detecting the multi-component complex,
wherein
a molecular weight of the small molecule is less than 5,000 Daltons; and
the small molecule binding partner is capable of specifically binding to the small molecules and has at least two antigen-binding sites, and is capable of binding to at least two small molecules to obtain the complex of small molecules/small molecule binding partner, and forming at least two new conformational epitopes distinct from the small molecule in a free state and the small molecule binding partner in a free state; and the first complex antibody and the second complex antibody each specifically bind to at least one new conformational epitope, and neither the first complex antibody nor the second complex antibody is an antibody against the small molecule antigen or a specific antibody against the small molecule binding partner.

For a long time, due to simple structures and small molecular weights of the small molecules, it is impossible to establish a non-competitive detection mode similar to a sandwich method. The present invention creatively obtains at least two new conformational epitopes by forming a complex of small molecules/small molecule binding partner, thereby enabling to construct a detection system based on a dual-complex antibody sandwich, and significantly improving specificity and sensitivity of a detection result.

As understood by those skilled in the art, small molecules are typically haptens, and can be organic substances such as organic compounds or short peptides.

Exemplary small molecules are any one of a drug, a biotoxin, an environmental pollutant, a hormone, or a metabolite.

The drugs can include pesticides, human drugs, and veterinary drugs. The drugs include those administered drugs for therapeutic purposes and those administered drugs for illegal purposes. A concept of drugs also broadly covers pharmaceutical intermediates or byproducts. Exemplary drugs include immunosuppressants such as FK506, CSA, digoxin, everolimus, and sirolimus.

The biotoxins can be substances produced by various organisms (including bacteria, fungi, plants, animals, etc.,) during growth and metabolism processes that are toxic to another organism. Exemplary biotoxins include aflatoxin, microcystin, and T2 toxin.

The environmental pollutants can be, such as, endocrine disruptors (EDCs), "three-causing" (carcinogenic, mutagenic, and reprotoxic) substances (CMRs), persistent organic pollutants (POPs), etc.

In some embodiments, the hormone is a thyroid hormone or a steroid hormone.

In some embodiments, the small molecule is any one selected from the group consisting of triiodothyronine, tetraiodothyronine, reverse triiodothyronine, cortisol, estradiol, estriol, testosterone, progesterone, 17α-hydroxyprogesterone (17α-OHP), androstenedione, and aldosterone.

In some embodiments, the small molecule is selected from metabolites such as 25-hydroxyvitamin D.

In some embodiments, the small molecule is selected from vitamin B₁₂, folic acid and other organic compounds, or from short peptides such as angiotensin I and angiotensin II.

In some embodiments, the complex of small molecules/small molecule binding partner is formed by contacting the small molecules with the small molecule binding partner in a free state.

In some embodiments, a complex antibody is defined as the first complex antibody or the second complex antibody; and an affinity constant between the complex antibody and the new conformational epitope to which it binds is K_{D1}, and the affinity constant between the complex antibody and the small molecule binding partner is K_{D2}, wherein K_{D2}/K_{D1} ≥ 10²:1, or ≥ 10³:1, or ≥ 10⁴:1. The above difference in affinity is beneficial to more specific binding of the first complex antibody and the second complex antibody to the new conformational epitope, improving detection sensitivity.

As mentioned above, it is easy to understood that the K_{D1} and K_{D2} of the first complex antibody and the K_{D1} and K_{D2} of the second complex antibody can be the same or different values, but K_{D2}/K_{D1} thereof all satisfy the above proportional relationship.

In some embodiments, an affinity constant between the small molecule binding partner and the small molecule is K_{D3} ≤ 9 × 10⁻⁹.

In some embodiments, K_{D1} ≤ 9 × 10⁻⁹.

In some embodiments, K_{D2} ≥ 1 × 10⁻⁶.

In some embodiments, 1×10⁻⁶ ≤ K_{D2}≤ 9×10⁻⁴, or 1×10⁻⁶ ≤ K_{D2} ≤ 9×10⁻⁵.

In some embodiments, the complex antibody does not bind or weakly binds to the small molecule in a free state.

An SPR method is used to detect the affinity constant in the present invention. A detection principle thereof is as follows: based on optical principles, when biomolecules bind or dissociate on a surface of a sensor chip, a change in a refractive index of the surface of the chip is caused, thereby leading to a change in an SPR angle. By monitoring such change, intermolecular interactions can be analyzed in real time.

An exemplary process mainly includes following steps: first, the anti-small-molecule antibody/complex antibody to be tested is immobilized on the surface of the sensor chip; then, a standard substance/anti-small-molecule antibody to be tested at different concentration gradients is used as a mobile phase to flow through the chip in sequence, and a dynamic change of the SPR angle during molecular binding and dissociation is monitored in real time to generate binding/dissociation curves; finally, the affinity constant (K_{D}) is calculated by fitting curve data with a software to evaluate the antibody affinity.

The anti-small-molecule antibody in a free state refers to the anti-small-molecule antibody that is not coupled to a solid phase or a signaling substance, and of course, is not bound to the small molecule.

In some embodiments, the method further includes pretreating the small molecule in a binding state to release the small molecule into a free state. Pretreatment is generally performed before obtaining the complex of small molecules/small molecule bonding partner. Since certain small molecules, especially hormone-based substances (typically such as T3 and/or T4 antigens), exist in a binding form bound to binding proteins in a certain proportion, pretreatment steps can be selectively added based on different detection requirements before a detection. The pretreatment step can be adding a pretreatment agent to a sample before the sample reacts with the small molecule binding partner when detecting the sample, causing the small molecules in the sample to dissociate from the binding substances (typically binding proteins) in the sample. Based on this, the present invention can not only determine total amount of an analyte, but also determine amount of an analyte in a free state, amount of an analyte in a binding state, a ratio of the analyte in a binding state to the analyte in a free state and/or total analyte, or a ratio of the analyte in a free state to the analyte in a binding state and/or total analyte.

In some embodiments, the method further includes: diluting the sample. Dilution is generally performed before obtaining the complex of small molecules/small molecule binding partner.

In some embodiments, the sample is reacted directly with the small molecule binding partner, the first complex antibody, and the second complex antibody without dilution.

The solution of the present invention can avoid the Hook effect, thus avoiding dilution in detecting some samples (e.g., high-value samples), thereby preventing interference from a matrix effect of the dilution solution on the detection and accelerating a detection speed.

In some embodiments, the first complex antibody and the second complex antibody have the same or different CDR sequences. In some embodiments, the first complex antibody and the second complex antibody are antibodies derived from the same monoclone.

In some embodiments, the small molecule binding partner is an anti-small-molecule antibody.

More advantageously, the anti-small-molecule antibody, the first complex antibody, and the second complex antibody are all monoclonal antibodies.

In some embodiments, the anti-small-molecule antibody is any one selected from the group consisting of IgG, IgA, IgM, IgD, and IgE.

In some embodiments, the anti-small-molecule antibody is an intact immunoglobulin or antibody fragment.

In some embodiments, the anti-small-molecule antibody includes two or more antibody variable regions, further optionally F(ab')₂. In some preferred embodiments, the anti-small-molecule antibody includes two or more antibody variable regions, wherein sequences of the two or more variable regions are identical or different.

In some embodiments, the first complex antibody or the second complex antibody is an intact immunoglobulin or antibody fragment.

In some embodiments, the first complex antibody or the second complex antibody includes one or more antibody variable regions, further optionally Fv, Fab, Fab', or F(ab')₂ fragments.

In some embodiments, the first complex antibody is conjugated to a solid phase.

The "solid phase" refers to a solid-phase material that interacts with a liquid-phase reagent through a heterogeneous reaction. Applications of the solid phase are well-known in the fields of chemistry, biochemistry, pharmacy, and molecular biology. Many types of solid phases have been developed according to the technical problems to be solved. Any of these can be used in the context of the present invention. For example, the solid phase used in the method of the present invention can include following components: silica, cellulose acetate, cellulose nitrate, nylon, polyester, polyether sulfone, polyolefins, or polyvinylidene fluoride, or combinations thereof. Other suitable solid phase includes, but is not limited to, controlled pore glass, glass plates or slides, magnetic spheres, polystyrene, and active dextran. In other aspects, synthetic organic polymers (such as polyacrylamide, polymethacrylate, and polystyrene) are also illustrative support surfaces. In addition, polysaccharides (such as cellulose and dextran) are other illustrative examples of the support surfaces. Other support surfaces, such as fibers, are also feasible.

Common resin-based solid phases used in, such as, combinatorial chemistry or protein chemistry, include: polystyrene resins (e.g., crosslinked with divinylbenzene); hydroxymethyl polystyrene; aminomethyl polystyrene; TentaGel resins (TG) and ArgoGel (AG); polystyrene/DVB poly(ethylene glycol) grafted copolymer (PS-PEG)-Bayer; crown/needle (CP) (radiation-grafted polyethylene/polypropylene support); diatomite/polyacrylamide-based resins (KPA); controlled pore glass; and PEGA-poly(ethylene glycol)/dimethacrylamide copolymer.

Immobilization on the solid phase can be achieved using following solid phase, and the solid phase is modified or activated to include functional groups that enable covalent coupling of the entity or support to the first complex antibody. The functional groups are one or more selected from the group consisting of carboxyl, amino, thiol, and toluenesulfonyl groups. The first complex antibody can also be non-covalently attached to a surface via, such as, ionic or hydrophobic mechanisms, and separated by a release agent that locally inhibits these mechanisms.

In one embodiment, the solid phase can be fibrous or granular, generally allowing for proper contact. A size of the solid phase suitable for the method of the present invention can vary depending on the chosen method. A capturing molecule can bind to only one solid phase (e.g., a porous plate) or to a plurality of solid phases (e.g., beads). In one embodiment, the solid phase can be fibrous or granular to allow for optimal contact. The size of the solid phase can vary and can be selected depending on the method to be performed.

In some embodiment, the solid phase is of a thin sheet, a pre-formed disc, a cylinder, a single fiber, or a solid support composed of particles.

In the present invention, a shape of a term "particle" (sometimes referred to as "bead", "microbead", or "microsphere") can generally vary. For example, in one particular embodiment, the particle is spherical. However, it should be understood that other shapes, such as plate-like, rod-like, disc-like, strip-like, tubular, or irregular shapes, can be also conceived in the present invention. In addition, the size of the particles can also vary. For example, an average size (e.g., diameter) range of the particles can be about 0.1 nm-1 mm, such as, 1 nm, 10 nm, 100 nm, 500 nm, 1 µm, 2 µm, 5 µm, 10 µm, 20 µm, 50 µm, 100 µm, or 500 µm, which is also the preferred diameter of particles.

In some embodiments, the particles are magnetic spheres, such as ferrosoferric oxide magnetic nanospheres.

In some embodiments, the second complex antibody is conjugated with a signaling substance.

The signaling substance refers to any substance capable of generating a signal used for a direct or indirect detection. Therefore, the signaling substance can be detected directly or indirectly. For the direct detection, the signaling substance suitable for the present invention can be selected from any known group of detectable markers. In some embodiments, the signaling substance is selected from the group consisting of a chromogen, a fluorescent group, a chemiluminescent group (e.g., isoluminol and its derivatives, acridinium ester or dioxetane), an electrochemiluminescent compound, a catalyst, an enzyme, an enzyme substrate, a dye, and a fluorescent dye (e.g., fluorescein, coumarin, rhodamine, oxazine, resorufin, cyanine and a derivative thereof). Other examples of the signaling substance include: a luminescent metal complexes such as a ruthenium or europium complexes (e.g., used for ECLIA), an enzyme (e.g., used for ELISA), and a radioisotope (e.g., used for RIA). In some embodiments, the signaling substance is selected from the group consisting of ABEI and a derivative thereof, an acridinium ester, alkaline phosphatase, and horseradish peroxidase.

Indirect detection systems include, such as, labeling the second complex antibody with the first partner of a bioaffinity binding pair. Examples of suitable binding pairs include: biotin or biotin analogues such as aminobiotin, iminobiotin, or dethiobiotin/avidin or streptavidin, sugar/lectin, and nucleic acids or nucleic acid analogues/complementary nucleic acids.

In some embodiments, the method includes: contacting a sample with the small molecule binding partner in a free state, followed by simultaneously or stepwise contacting the first complex antibody conjugated to the solid phase and the second complex antibody coupled with the signaling substance to allow for a sufficient reaction, so as to form a multi-component complex including "first complex antibody - complex of small molecules/small molecule binding partner - second complex antibody", and detecting the small molecule in the sample by detecting the multi-component complex.

In some embodiments, the method includes following steps that:
a) the small molecule is contacted with a small molecule binding partner in a free state to form a complex of small molecules/small molecule binding partner;
b) the complex of small molecules/small molecule binding partner, the first complex antibody conjugated to the solid phase, and the second complex antibody coupled with the signaling substance are simultaneously or stepwise contacted, and the system is incubated for a sufficient reaction to obtain the multi-component complex; and
c) the small molecule is determined by the signaling substance.

Determination of the present invention are typically performed under conventional conditions used for such determination, such as, at a temperature of 4 °C - 45 °C. More typically, the temperature is 25 °C - 45 °C, such as, 30 °C, 35 °C, 36 °C, 37 °C, 38 °C, 39 °C, and 40 °C. The determination is typically performed in an aqueous solution; and a pH is 5 - 9, such as, a pH of 6, a pH of 7, or a pH of 8.

In some embodiments, step b) further includes at least one washing step to separate unbound substances from the solid phase.

The unbound substances primarily include impurities in the sample that have not bound to the solid phase, the small molecule binding partner in a free state in the system that has not bound to the small molecule, and the second complex antibody coupled with signaling substances that has not participated in forming a multi-component complex.

In some embodiments, a two-step washing process can be used, with the first washing step performed after contacting and incubating the complex of small molecules/small molecule binding partner with the first complex antibody conjugated to the solid phase until the two have reacted sufficiently, thereby separating a first unbound substance from the solid phase. The first unbound substance primarily includes impurities in the sample that have not bound to the solid phase, and the small molecule binding partners in a free state in the system that have not bound to small molecules. The second washing step is performed after contacting the second complex antibody coupled with the signaling substance with the solid phase obtained in the first washing step and incubating until the two are fully bound to form a multi-component complex, thereby separating a second unbound substance from the multi-component complex. The second unbound substance primarily includes the second complex antibody coupled with signaling substances that has not participated in forming a multi-component complex.

In some embodiments, a one-step washing process can be used, and performed only after the complex of small molecules/small molecule binding partner has been contacted with the first complex antibody conjugated to the solid phase, and the second complex antibody coupled with the signaling substance and the system is incubated until sufficient binding to form a multi-component complex, allowing the above-mentioned unbound substances to be separated from the system at one time during this washing step.

In some other embodiments, the method is a lateral flow assay. When a lateral flow assay is used for detection, advantageously, the solid phase is a solid phase in a strip shape, which is typically provided with a "detection line" and a "control line".

According to the second aspect of the present invention, the present invention further relates to a kit, including the first complex antibody, the small molecule binding partner, and the second complex antibody defined in the first aspect above.

In some embodiments, the small molecule binding partner is present in a free state in the assay kit, the first complex antibody is conjugated to the solid phase, and the second complex antibody is coupled with the signaling substance.

The kit can further include one or more containers, and the container can include a dilution buffer, a reaction solution, a chromogenic reagent, etc. The kit can further include an instruction for use.

The kit can be used for diagnosis or prognosis of a disease or condition. For example, biomarkers (e.g., hormones) indicating specific diseases, conditions, or prognosis can be determined within the context of the present invention. Specifically, the kit and the method of the present invention can be used for individual diagnosis, prognosis, risk assessment, risk stratification, monitoring, and/or treatment control. A term "individual" herein refers to a living human or non-human animal, such as a mammal, particularly a human. An individual is most preferably a patient. As used herein, a term "patient" refers to a living human or non-human animal (usually human) who is receiving medical care or should receive medical care due to a disease or medical condition. This includes individuals without a clearly defined disease and undergoing examination for pathological indexes. Therefore, the methods and the assays herein are applicable to diseases of human and animal.

In some embodiments, the kit further includes a solid phase. In some embodiments, the solid phase is the solid phase defined in the first aspect above.

In some embodiments, the first complex antibody is conjugated to the solid phase.

In some embodiments, the second complex antibody is coupled with a signaling substance. In some embodiments, the signaling substance is the signaling substance as defined in the first aspect above.

In some embodiments, the small molecule binding partner is in a free state, that is, a state in which it has not formed a complex with the small molecule.

The embodiments of the present invention will be described in detail below with reference to examples. It should be understood that these examples are only used to illustrate the present invention and are not intended to limit the scope of the present invention. For experimental methods without specific conditions in following examples, guidelines given in the present invention shall be referred to first, or experimental manuals or conventional conditions in the art can be followed, or other experimental methods known in the art can be referred to, or conditions recommended by the manufacturer can be followed.

In specific examples below, measurement parameters involving raw material components may have slight deviations within a weighing accuracy range unless otherwise specified. Parameters involves temperature and time, allowing acceptable deviations caused by instrument testing accuracy or operational accuracy.

### Example 1 Preparation of antibody

### 1. Sources of anti-small-molecule antibodies

Source of cortisol antibody: Snibe cortisol competitive assay kit (Catalog No.: 130270002M, Batch No.: 468240111).

Source of 17α-OHP antibody: Snibe 17α-OHP competitive assay kit (Catalog No.: 130201002M, Batch No.: 026240112).

### 2. Preparation process of complex antibody

### 1) Preparation of complex antibody of cortisol/cortisol antibody

### Immunization of mice

Both a natural standard of cortisol (Sigma) and the above-mentioned cortisol antibody were dialyzed into 0.01M PBS and incubated at a molar ratio of 20:1 at 37 °C for 15 min to form a mixture including a complex of cortisol/cortisol antibody, as an immunogen.

By using an equal volume of Freund's complete adjuvant (Sigma), the mixture was emulsified into water-in-oil droplets. 6-8-week-old SPF-grade Balb/c mice were subcutaneously injected at a plurality of sites with 200 µg/mouse. After a 3-week interval, the antigen was emulsified with the Freund's incomplete adjuvant and the mice were subcutaneously injected at a plurality of sites with 100 µg/mouse. Two booster immunizations were administered. An intraperitoneal injection was administered three days before fusion for a booster.

### b) Fusion and cloning

Spleens were collected from immunized mice, and dispersed single spleen cells were obtained by grinding and separation. Spleen cells were fused with myeloma cells using polyethylene glycol according to a standard procedure. After fusion, the cells were cultured for one week, followed by changing medium. Supernatant was collected the next day for indirect enzyme immunoassay detection.

### c) Detection method as follows.

Pepsin digestion treatment was performed on cortisol antibodies to obtain the F(ab')₂ fragment as a coating antigen. After blocking, PBS or a natural cortisol standard (Sigma) was added, followed by adding cell supernatant. Goat anti-mouse IgG Fcy-HRP was used as a chromogenic agent. Positive wells showing no or low reactivity with the cortisol antibody F(ab')₂ fragment but strong reactivity with the complex were selected. Further competitive method detection was performed on the positive wells: using cortisol-bovine IgG as the coating antigen, after blocking, PBS or the natural cortisol standard was added, followed by adding cell supernatant. Goat anti-mouse IgG Fcy-HRP was used as the chromogenic agent. Positive wells showing no competitive inhibition on cortisol were selected. Subcloning was further performed using a limiting dilution method. After one week of culture, enzyme immunoassay was continued and the detection method was the same as above. Positive wells were selected for limiting dilution, which is repeated 3-4 times until all detected wells were positive and the cells in the wells formed single colonies. Cells from the optimal positive wells were selected for scale-up culture, yielding a specific hybridoma cell line, namely an anti-complex antibody.

### d) Preservation of positive cell lines

The hybridoma cell line was frozen in freezing medium (50% 1640, 40% FCS, and 10% DMSO) at -80 °C using a cell cooling box (cooling rate of -1 °C/minute) (Corning), followed by transferring to liquid nitrogen for preservation after 24 h.

### e) Preparation of ascites from positive cell lines

Positive cell lines were scale-up cultured, then injected into mice pre-vaccinated with IFA to prepare ascites. The ascites was collected to obtain anti-complex antibodies, which were subjected to affinity purification using the SPA method.

### 2) Preparation of complex antibody of 17α-OHP/17α-OHP antibody

A preparation method was similar to that used for the preparation of the above complex antibody of cortisol/cortisol antibody.

### 3. Binding characteristics of complex antibodies with complexes, anti-small-molecule antibodies, and antigens in a free state, and evaluation of binding affinity

Detection steps of evaluation of binding affinity of cortisol complex antibodies were as follows.
a. Detection of affinity between complex antibodies and small molecules or anti-small-molecule antibodies
   (1) Immobilization of complex antibody: ligand immobilization was performed using a direct amine coupling method. First, EDC/NHS (prepared immediately before use, and mixed at 1:1) was fed for 5-10 min to activate carboxyl groups; then, the complex antibody was diluted to 10 µg/ml with sodium acetate buffer at a pH of 4.5 and fed into a detection channel to a target coupling level (for small molecule analyte, high coupling, 20000 RU; and for large analyte, low coupling, 100-500 RU); then, ethanolamine (pH of 8.5) was fed for 5-10 min to block unreacted sites; and finally, equilibration buffer was used to wash until a baseline stabilized.
   (2) Detection of analyte: the small molecules were diluted to 100 µM, or the anti-small-molecule antibodies were diluted to 10 µM, followed by a 2-fold dilution to prepare 6 gradients, plus a zero concentration, resulting in a total of 8 concentrations. A multi-cycle kinetic detection method was used, with feeding sequentially from low concentration to high concentration at a flow rate of 10 µL/min for 180 s. A duration of dissociation was 600 s, and regeneration was performed using 10 mM Gly-HCl (pH of 2.0) for 120 s.
b. Detection of affinity between complex antibody and small molecule/anti-small-molecule antibody
   (1) Immobilization of anti-small-molecule antibody: ligand immobilization was performed using the direct amine coupling method. First, EDC/NHS (prepared immediately before use, and mixed at 1:1) was fed for 5-10 min to activate the carboxyl groups. Then, the anti-small-molecule antibody was diluted to 10 µg/ml with sodium acetate buffer at a pH of 4.5 and fed into the detection channel to a target coupling level (for large analyte, low coupling, 100-500 RU); then, ethanolamine (pH of 8.5) was fed for 5-10 min to block unreacted sites; and equilibration buffer was used to wash until a baseline stabilized.
   (2) Feeding of small molecule: the small molecule solution was diluted to 100 µM and fed until the curve stabilized.
   (3) Detection of co-feeding of small molecule and complex antibody: the complex antibody was diluted to 10 µM (containing 100 µM of small molecule), followed by a 2-fold dilution to prepare 6 gradients of concentrations (with diluent containing 100 µM of small molecule). A multi-cycle kinetic detection method was used, with feeding sequentially from low concentration to high concentration at a flow rate of 10 µL/min for 120 s. 100 µM small molecule solution was still used in a dissociation phase, followed by regeneration and baseline equilibration.
c. Data fitting and analysis were performed using an analytical software to calculate affinity.

Evaluation of binding affinity of 17α complex antibody was performed similarly to the above steps.

Detection results were as follows.

| | Complex Antibody No. | Anti-Small-Molecule Antibody | Small Molecule/Anti-Small-Molecule Antibody Complex |
|---|---|---|---|
| Cortisol | CS-S1-1 | 2.24*10⁻⁵ | 1.01*10⁻¹⁰ |
| | CS-S1-2 | 2.42*10⁻⁵ | 4.08*10⁻¹⁰ |
| 17α-OHP | 17α-OHP-S1-1 | 4.53*10⁻⁵ | 3.29*10⁻¹⁰ |
| | 17α-OHP-S1-2 | 5.02*10⁻⁵ | 4.14*10⁻¹⁰ |

Data indicate that the complex antibody does not bind to the small molecules in a free state, exhibits weak binding affinity to the anti-small-molecule antibodies that are not bound to small molecules, and shows high affinity for the complex formed by the anti-small-molecule antibody and the small molecule.

### Example 2 Preparation and performance validation of cortisol kit

### 1. Components of cortisol kit

### 1) Components of a first-generation small molecule sandwich assay kit

Buffer: PBS buffer containing 0.25% ANS.

Solid-phase complex antibody reagent: TRIS buffer containing 0.2% NaN₃, and complex antibody specifically binding to cortisol/cortisol antibody complex and linked to solid phase.

Marked anti-small-molecule antibody reagent: PBS buffer containing 0.2% NaN₃, and cortisol antibody linked to signaling substance.

### 2) Components of a second-generation small molecule sandwich assay kit

Pre-reaction solution 1: TRIS buffer containing 0.25% ANS, and cortisol antibody.

Solid-phase first complex antibody reagent: TRIS buffer containing 0.2% NaN₃, and complex antibody linked to solid phase and specifically binding to cortisol/cortisol antibody complex.

Marked second complex antibody reagent: PBS buffer containing 0.2% NaN₃, and complex antibody linked to signaling substance and specifically binding to cortisol/cortisol antibody complex.

Specific antibody information is as follows.

| | Pre-Reaction Solution | Solid Phase Reagent | Marked Reagent |
|---|---|---|---|
| Competitive Assay Kit | Snibe Cortisol Competitive Assay Kit (Catalog No.: 130270002M, Batch No.: 468240111) | | |
| First-Generation Small Molecule Sandwich Assay Kit | - | CS-S1-1 (9 µg/mL) | Cortisol Antibody (1 µg/mL) |
| Example 2.1 | Cortisol Antibody (2.5 µg/mL) | CS-S1-1 (8µg/mL) | CS-S1-1 (0.5µg/mL) |
| Example 2.2 | Cortisol Antibody (2.5 µg/mL) | CS-S1-1 (8µg/mL) | CS-S1-2 (0.5µg/mL) |

### 2. Detection process:

### 1) Detection process of first-generation small molecule sandwich method

① 10 µL of sample, 150 µL of marked anti-small-molecule antibody reagent, and 20 µL of solid-phase complex antibody reagent were added to 80 µL of Buffer, mixed well and incubated at 37 °C for 4 min. Supernatant was removed by washing.
② A substrate solution was added and optical signal was detected.

Detection process of cortisol kit-related examples (one-step washing method)
① 10 µL of sample was added to 100 µL of pre-reaction solution, mixed well and incubated at 37 °C for 4 min.
② 150 µL of marked second complex antibody reagent and 20 µL of solid-phase first complex antibody reagent were added in step ①, mixed well and incubated at 37 °C for 4 min. Supernatant was removed by washing.
③ A substrate solution was added and optical signal was detected.

### 3) Detection process of cortisol kit-related examples (two-step washing method)

Detection steps included:
①10 µL of sample was added to 100 µL of pre-reaction solution, mixed well and incubated at 37 °C for 4 min;
② 20 µL of solid-phase first complex antibody reagent was added in step , mixed well, and incubated at 37 °C for 4 min, then supernatant was removed by washing;
③ 150 µL of marked second complex antibody reagent was added to step , mixed well and incubated at 37 °C for 4 min, then supernatant was removed by washing; and
④ a substrate solution was added and optical signal was detected.

### Validation results of sensitivity, discrimination, and Hook effect

Detection instrument: MAGLUMI X8
Preparation of sample: a cortisol (Sigma) standard mother solution was prepared using DMSO. During detection, PBS diluent was used to prepare cortisol standards (Sigma) with gradient working concentrations of 0 ng/mL (point A), 10 ng/mL (point B), 16.683 ng/mL (point C), 27.832 ng/mL (point D), 46.431 ng/mL (point E), 77.46 ng/mL (point F), 129.224 ng/mL (point G), 215.583 ng/mL (point H), 359.625 ng/mL (point I), and 600 ng/mL (point J). Evaluation results are as follows.

| **Ten-Point Curve** | Competitive Method | | First-Generation Small Molecule Sandwich Method | | Example 2.1 (One-Step Washing) | | Example 2.2 (One-Step Washing) | | Example 2.1 (Two-Step Washing) | |
|---|---|---|---|---|---|---|---|---|---|---|
| **A (PBS-1)** | 1775363 | / | 7607 | | 4255 | / | 4184 | / | 4494 | / |
| **10 ng/ml** | 1011889 | 1.75 | 120751 | 15.87 | 17615 | 4.14 | 21045 | 5.03 | 20403 | 4.54 |
| **16.683 ng/ml** | 779927 | 1.30 | 183541 | 1.52 | 37329 | 2.12 | 48824 | 2.32 | 41213 | 2.02 |
| **27.832 ng/ml** | 555945 | 1.40 | 275312 | 1.50 | 76524 | 2.05 | 109854 | 2.25 | 79130 | 1.92 |
| **46.431 ng/ml** | 376207 | 1.48 | 390944 | 1.42 | 141569 | 1.85 | 223003 | 2.03 | 139268 | 1.76 |
| **77.46 ng/ml** | 262013 | 1.44 | 539502 | 1.38 | 253408 | 1.79 | 414785 | 1.86 | 238149 | 1.71 |
| **129.224 ng/ml** | 170704 | 1.53 | 717537 | 1.33 | 433327 | 1.71 | 725874 | 1.75 | 385802 | 1.62 |
| **215.583 ng/ml** | 109960 | 1.55 | 868220 | 1.21 | 706323 | 1.63 | 1212209 | 1.67 | 609568 | 1.58 |
| **359.625 ng/ml** | 67724 | 1.62 | 1024517 | 1.18 | 1130116 | 1.60 | 2016142 | 1.66 | 944830 | 1.55 |
| **600 ng/ml** | 42447 | 1.60 | 1126969 | 1.10 | 1706475 | 1.51 | 3266151 | 1.62 | 1424894 | 1.51 |
| **A/B (B/A)** | 1.75 | | 15.87 | | 4.14 | | 5.03 | | 4.54 | |
| **A/J (J/A)** | 41.83 | | 148.15 | | 401.05 | | 780.63 | | 317.06 | |

Compared to commercially available competitive methods, the sensitivity and discrimination of the first-generation sandwich method and various examples are significantly improved, exhibiting relatively obvious advantages. Specifically, in the various examples, that is, when using a scheme of a dual-complex antibody sandwich method to detect cortisol, a value of B/A is significantly higher than that of an assay kit of commercially available competitive method, that is to say, the sensitivity of the examples provided by the present invention is improved compared to that of the competition method. Further, a value of J/A is higher than those of a kit of commercially available competitive method and a kit of the first-generation sandwich method, indicating that the examples provided by the present invention have better discrimination in samples with a large-span concentration gradient. Further, for high-value samples (sample at point I and sample at point J), a ratio of optical signals thereof approaches a concentration ratio of samples at point I and point J, proving that the Hook effect is effectively solved in high-value samples. Therefore, in the various examples provided by the present invention, the complex antibodies can be selected from either the same complex antibody or different complex antibodies, and the detection process can select a scheme of the one-step washing method or the two-step washing method, all of which exhibit significant advantages in the detection of cortisol.

### (I) Validation of detection capability for plasma samples

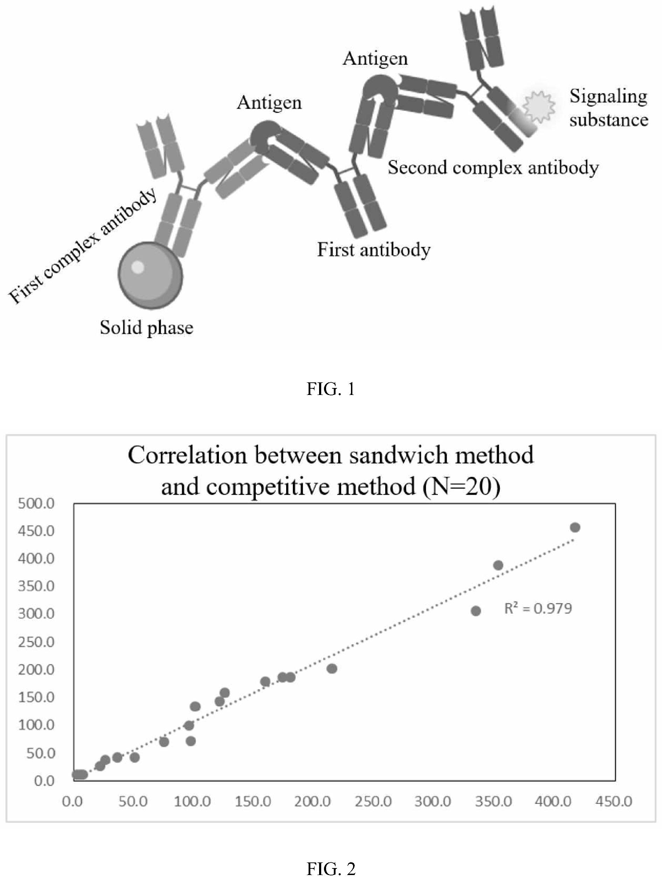

Twenty plasma samples were tested using the kit in Example 2.1 above and the detection process of the one-step washing method, and detection results were collected, along with detection results of the same blood samples obtained using a commercially available competitive method kit. Correlation curves were plotted, as shown in FIG. 2.

In detecting the blood sample, the sandwich method shows a correlation with the competitive method, indicating that the scheme is applicable to detect an actual sample and has reliability.

### (II) Validation of specificity performance

Detection instrument: MAGLUMI X8
Preparation of sample: mother solutions of cortisol analogues (fludrocortisone, dexamethasone, prednisolone, and corticosterone) were prepared using DMSO. During detection, the four main analogues were prepared with PBS diluent to working concentrations of 1 µg/mL, 0.1 µg/mL, 1 µg/mL, and 0.1 µg/mL, respectively. Following detection steps were used for evaluation.

Samples containing the above analogues were tested using the kit in Example 2.1 and the detection process of the one-step washing method. Detection results are as follows.

| Main Analogues | Detection Concentration of Analogues µg/mL | Cross-Rate of Analogues in Dual-Complex Sandwich Method (%) | Cross-Rate of Analogues in Competitive Method Kit (%) |
|---|---|---|---|
| Fludrocortisone | 1 | 1.15 | 35.7 |
| Dexamethasone | 0.1 | 0.50 | 0.60 |
| Prednisolone | 1 | 3.26 | 13.4 |
| Corticosterone | 0.1 | 0.20 | 1.2 |

As can be seen from data, the dual-complex antibody sandwich method exhibits relatively good specificity when used to detect cortisol. Since the complex antibody has a high specificity for the complex formed by anti-small-molecule antibody and small molecules, use of two complex antibodies further improves the specificity of the entire detection system.

### Example 3 Preparation and performance validation of 17α-OHP kit

### 1. Components of 17α-OHP kit

### 1) Components of first-generation small molecule sandwich assay kit

Buffer: PBS buffer.
Solid-phase anti-small-molecule antibody reagent: PBS buffer containing 0.2% NaN₃, and 17α-OHP antibody linked to solid phase.
Marked complex antibody reagent: TRIS-HCl buffer containing 0.2% NaN₃, and complex antibody linked to the signaling substance and specifically binding to 17α-OHP/17α-OHP antibody complex.

### 2) Components of second-generation small molecule sandwich assay kit

Pre-reaction solution 1: TRIS buffer, and 17α-OHP antibody.
Solid-phase first complex antibody reagent: PBS buffer containing 0.2% NaN₃, and complex antibody linked to solid phase and specifically binding to 17α-OHP/17α-OHP antibody complex.
Marked second complex antibody reagent: TRIS-HCl buffer containing 0.2% NaN₃, and complex antibody linked to solid phase and specifically binding to 17α-OHP/17α-OHP antibody complex.

Specific antibody information is as follows.

| | Pre-Reaction Solution | Solid Phase Reagent | Marked Reagent |
|---|---|---|---|
| Competitive Assay Kit | Snibe 17α-OHP Competitive Assay Kit, Catalog No.: 130201002M | | |
| First-Generation Small Molecule Sandwich Assay Kit | - | 17α-OHP Antibody (6 µg/mL) | 17α-OHP -S1-1 (0.2 µg/mL) |
| Example 3.1 | 17α-OHP Antibody (0.5 µg/mL) | 17α-OHP-S1-1 (8 µg/mL) | 17α-OHP-S1-2 (0.25 µg/mL) |
| Example 3.2 | 17α-OHP Antibody (0.5 µg/mL) | 17α-OHP-S1-1 (8 µg/mL) | 17α-OHP-S1-1 (0.25 µg/mL) |

### 2. Detection process:

### 1) Detection process of first-generation small molecule sandwich method

① 10 µL of sample and 20 µL of solid-phase anti-small-molecule antibody reagent were added to 100 µL of Buffer, mixed well, and incubated at 37 °C for 5 min. Supernatant was removed by washing.
② 100 µL of marked complex antibody reagent was added in step ①, mixed well, and incubated at 37 °C for 5 min. Supernatant was removed by washing.
③ A substrate solution was added and optical signal was detected.

Detection process of 17α-OHP kit-related examples (one-step washing method)
① 10 µL of sample and 20 µL of solid-phase first complex antibody reagent were added to 100 µL of pre-reaction solution, mixed well and incubated at 37 °C for 5min.
160 µL of marked second complex antibody reagent was added in step ①, mixed well and incubated at 37 °C for 5min. Supernatant was removed by washing.
③ A substrate solution was added and optical signal was detected.

### (I) Validation results of sensitivity, discrimination, and Hook effect

Detection instrument: MAGLUMI X8
Preparation of sample: 17α-OHP standard (J&K Scientific) mother solution was prepared using DMSO. During detection, PBS diluent was used to prepare 17α-OHP standards (J&K Scientific) with gradient working concentrations of 0 ng/mL (point A), 0.1 ng/mL (point B), 0.204 ng/mL (point C), 0.416 ng/mL (point D), 0.849 ng/mL (point E), 1.732 ng/mL (point F), 3.534 ng/mL (point G), 7.209 ng/mL (point H), 14.706 ng/mL (point I), and 30 ng/mL (point J). Evaluation results are as follows.

| **Ten-Point Curve** | Competitive Method | | First-Generation Sandwich Method | | Example 3.1 (Cross-Pairing) | | Example 3.2 (Self-Pairing) | |
|---|---|---|---|---|---|---|---|---|
| **A (PBS-1)** | 121843 1 | / | 8104 | / | 1184 | / | 1227 | / |
| **0.1 ng/ml** | 117742 2 | 1.03 | 11904 | 1.47 | 1906 | 1.61 | 1755 | 1.43 |
| **0.204 ng/ml** | 114147 9 | 1.03 | 19206 | 1.61 | 2935 | 1.54 | 2544 | 1.45 |
| **0.416 ng/ml** | 101372 1 | 1.13 | 32650 | 1.70 | 4579 | 1.56 | 3841 | 1.51 |
| **0.849 ng/ml** | 809557 | 1.25 | 51261 | 1.57 | 7371 | 1.61 | 6185 | 1.61 |
| **1.732 ng/ml** | 637193 | 1.27 | 73816 | 1.44 | 12679 | 1.72 | 10700 | 1.73 |
| **3.534 ng/ml** | 474153 | 1.34 | 101128 | 1.37 | 23076 | 1.82 | 19367 | 1.81 |
| **7.209 ng/ml** | 318155 | 1.49 | 127421 | 1.26 | 40152 | 1.74 | 33506 | 1.73 |
| **14.706 ng/ml** | 207121 | 1.54 | 142712 | 1.12 | 67455 | 1.68 | 54279 | 1.62 |
| **30 ng/ml** | 129450 | 1.60 | 164119 | 1.15 | 110627 | 1.64 | 83851 | 1.54 |
| **A/B (B/A)** | 1.03 | | 1.47 | | 1.61 | | 1.43 | |
| **A/J (J/A)** | 9.41 | | 20.25 | | 93.43 | | 68.34 | |

As can be seen from the validation results, in the various examples provided by the present invention, the dual-complex antibody sandwich method is used by using complex antibody that can be selected from the same complex antibody or different complex antibodies for detecting 17-αOHP, demonstrating a good effect on improving performance of sensitivity and discrimination of detection, as well as the Hook effect of a high-value sample.

### (II) Validation results of specificity

Detection instrument: MAGLUMI X8
Preparation of sample: mother solutions of 17α-OHP analogues (progesterone, 17α-hydroxypregnenolone, 11-deoxycortisol, and 21-deoxycortisol) were prepared using DMSO. During detection, PBS diluent was used to prepare the four major analogues with working concentrations of 100 ng/mL, 100 ng/mL, 20 ng/mL, and 20 ng/mL, respectively. Following detection steps were used for evaluation.

Samples containing the above analogues were tested using the kit in Example 3.1 and the detection process of the one-step washing method. Detection results are as follows.

Detection results are as follows.

| Major Analogues | Detection Concentration of Analogues ng/mL | Cross-rate of Analogues in Dual-Complex Sandwich Method % | Cross-Rate of Analogues in Competitive Method Kit % |
|---|---|---|---|
| progesterone | 100 | 1.0 | 4.2 |
| 17α-hydroxypregnenolone | 100 | 11.2 | 33.5 |
| 11-deoxycortisol | 20 | 2.6 | 15.2 |
| 21-deoxycortisol | 20 | 1.4 | 13.0 |

As can be seen from data, the dual-complex antibody sandwich method exhibits relatively good specificity when used to detect 17α-OHP. Since the complex antibody has a high specificity for the complex formed by the anti-small-molecule antibody and the small molecules, use of two complex antibodies further improves the specificity of the entire detection system.

The above examples only illustrate several embodiments of the present invention, and descriptions thereof are relatively specific and detailed, but they should not be construed as limitation on the scope of the present invention. It should be noted that those ordinarily skilled in the art can make various modifications and improvements without deviating from the concept of the present invention, and these all fall within the scope of protection of the present invention. Therefore, the scope of protection of the present invention should be determined by the appended claims, and a specification and drawings can be used to interpret the content of the claims.

## Claims

1. A method for detecting a small molecule, **characterized by** comprising: forming a multi-component complex comprising a "first complex antibody - complex of small molecules/small molecule binding partner - second complex antibody", and detecting the small molecules in a sample by detecting the multi-component complex,
wherein a molecular weight of the small molecule is less than 5,000 Daltons; and
the small molecule binding partner is capable of specifically binding to the small molecules and has at least two antigen-binding sites, and is capable of binding to at least two small molecules to obtain the complex of small molecules/small molecule binding partner, and forming at least two new conformational epitopes distinct from the small molecules in a free state and the small molecule binding partner in a free state; the first complex antibody and the second complex antibody each specifically bind to at least one new conformational epitope, and neither the first complex antibody nor the second complex antibody is an antibody against an antigen of the small molecules or a specific antibody against the small molecule binding partner.

2. The method according to claim 1, wherein the molecular weight of the small molecules is less than 3,000 Daltons; optionally, the molecular weight is less than 2,000 Daltons; optionally, the molecular weight is less than 1,500 Daltons; optionally, the molecular weight is less than 1,000 Daltons;
optionally, the small molecules are any one selected from the group consisting of a drug, a biotoxin, an environmental pollutant, a hormone, or a metabolite;
optionally, the hormone is a thyroid hormone or a steroid hormone; optionally, the small molecules are any one selected from the group consisting of triiodothyronine, tetraiodothyronine, reverse triiodothyronine, cortisol, estradiol, estriol, testosterone, progesterone, 17α-hydroxyprogesterone, androstenedione, and aldosterone; and
optionally, the complex of small molecules/small molecule binding partner is formed by contacting the small molecules with the small molecule binding partner in the free state; optionally, the method further comprises: pretreating the small molecules in a binding state to release the small molecules into the free state; and optionally, the method further comprises: diluting the sample.

3. The method according to claim 1, wherein a complex antibody is defined as the first complex antibody or the second complex antibody; an affinity constant between the complex antibody and the new conformational epitope to which the complex antibody binds is K_{D1}, and an affinity constant between the complex antibody and the small molecule binding partner is K_{D2}, then K_{D2}/K_{D1} ≥ 10²:1, or ≥ 10³:1, or ≥ 10⁴:1;
optionally, an affinity constant between the small molecule binding partner and the small molecules is K_{D3} ≤ 9 × 10⁻⁹;
optionally, K_{D1} ≤ 9 × 10⁻⁹; optionally, K_{D2} ≥ 1 × 10⁻⁶; and
optionally, the first complex antibody and the second complex antibody have the same or different CDR sequences.

4. The method according to claim 1, wherein the small molecule binding partner is an anti-small-molecule antibody;
optionally, the anti-small-molecule antibody is any one selected from the group consisting of IgG, IgA, IgM, IgD, and IgE;
optionally, the anti-small-molecule antibody is an intact immunoglobulin or antibody fragment;
optionally, the anti-small-molecule antibody comprises two or more antibody variable regions, further optionally F(ab')₂; and
optionally, the anti-small-molecule antibody comprises two or more antibody variable regions, wherein sequences of the two or more variable regions are identical or different.

5. The method according to claim 1, wherein the first complex antibody or the second complex antibody is an intact immunoglobulin or antibody fragment; and optionally, the first complex antibody or the second complex antibody comprises one or more antibody variable regions, further optionally Fv, Fab, Fab', or F(ab')₂ fragments.

6. The method according to any one of claims 1 to 5, wherein the first complex antibody is conjugated to a solid phase; and
optionally, the solid phase is of a thin sheet, a pre-formed disc, a cylinder, a single fiber, or a solid support composed of particles; preferably, the particles are magnetic spheres.

7. The method according to claim 6, wherein the second complex antibody is coupled with a signaling substance;
optionally, the signaling substance is selected from the group consisting of a chromogen, a fluorescent group, a chemiluminescent group including acridinium ester or dioxetane, an electrochemiluminescent compound, a catalyst, an enzyme, an enzyme substrate, a dye, and a fluorescent dye including fluorescein, coumarin, rhodamine, oxazine, resorufin, cyanine and derivatives thereof; and
optionally, the signaling substance is selected from the group consisting of ABEI and derivatives thereof, an acridinium ester, alkaline phosphatase, and horseradish peroxidase.

8. The method according to claim 7, comprising:
a) contacting the small molecules with the small molecule binding partner in the free state to form the complex of small molecules/small molecule binding partner;
b) simultaneously or stepwise contacting the complex of small molecules/small molecule binding partner, the first complex antibody conjugated to the solid phase, and the second complex antibody coupled with the signaling substance, and incubating until a sufficient reaction to obtain the multi-component complex; and
c) determining the small molecules by the signaling substance, wherein
optionally, step b) further comprises at least one washing step to separate unbound substances from the solid phase.

9. A kit, **characterized by** comprising the first complex antibody, the small molecule binding partner, and the second complex antibody defined in the method according to any one of claims 1 to 8.

10. The kit according to claim 9, further comprising a solid phase, wherein
optionally, the first complex antibody is conjugated to the solid phase;
optionally, the solid phase is of a thin sheet, a pre-formed disc, a cylinder, a single fiber, or a solid support composed of particles; preferably, the particles are magnetic spheres;
optionally, the second complex antibody is coupled with a signaling substance;
optionally, the signaling substance is selected from the group consisting of a chromogen, a fluorescent group, a chemiluminescent group, an electrochemiluminescent compound, a catalyst, an enzyme, an enzyme substrate, a dye, and a fluorescent dye including fluorescein, coumarin, rhodamine, oxazine, resorufin, cyanine and derivatives thereof; and
optionally, the signaling substance is selected from the group consisting of ABEI and derivatives thereof, an acridinium ester, alkaline phosphatase, and horseradish peroxidase; and optionally, the small molecule binding partner is in the free state.
